# FASCICULE DE BREVET EUROPEEN

(11) **EP 1 931 621 B1**
(45) Date de publication et mention de la délivrance du brevet: **04.08.2010**
(21) Numéro de dépôt: 06831232.1
(22) Date de dépôt: 27.09.2006
(51) Int. Cl.: C07C 245/08, A61F 2/16, C09B 56/08, C08J 3/20

(54) **COMPOSES CONVENANT A TITRE DE COLORANTS JAUNES POLYMERISABLES ; COMPOSITIONS POLYMERISABLES ET/OU RETICULABLES, MATRICES POLYMERES ET LENTILLES INTRAOCULAIRES LES RENFERMANT**
ALS POLYMERISIERBARE GELBE FARBSTOFFE GEEIGNETE VERBINDUNGEN, POLYMERISIERBARE UND/ODER VERNETZBARE ZUSAMMENSETZUNGEN, POLYMERMATRIZEN UND, DIESE ENTHALTENDE INTRAOKULÄRE LINSEN
COMPOUNDS SUITABLE AS POLYMERIZABLE YELLOW DYES; POLYMERIZABLE AND/OR CROSSLINKABLE COMPOSITIONS, POLYMER MATRICES AND INTRAOCULAR LENSES CONTAINING SAME

(30) Priorité: 28.09.2005 FR 0509882
(43) Date de publication de la demande: 18.06.2008
(73) Titulaire: Corneal Innovation, 75002 Paris (FR)
(72) Inventeur: GOUSSÉ, Cécile, 74230 Dingy Saint Clair (FR)
(74) Mandataire: Le Roux, Martine
(86) Numéro de dépôt international: PCT/FR2006/050945
(87) Numéro de publication internationale: WO 2007/036672

(56) Documents cités:
- US-A- 5 470 932
- US-A- 5 496 899
- US-B1- 6 242 551

## Description

La présente invention concerne principalement des composés, convenant à titre de colorants jaunes polymérisables et leur utilisation, pour colorer plus particulièrement des lentilles intraoculaires.

La présente invention a plus précisément pour objet :
- de nouveaux composés : des dérivés fonctionnalisés, polymérisables, du produit colorant jaune, connu sous la dénomination C.I. Disperse Yellow 3, répertorié n° CAS 2832-40-8 (il s'agit du 4-(2-hydroxy-5-méthylphénylazo)acétanilide);
- la préparation desdits nouveaux composés ;
- leur utilisation à titre de colorants jaunes ;
- des compositions polymérisables et/ou réticulables les renfermant ;
- des matrices polymères colorées, susceptibles d'être obtenues à partir de telles compositions ; et, enfin,
- des lentilles intraoculaires, constituées, en totalité ou en partie, de telles matrices polymères colorées.

La présente invention a été développée dans le contexte du traitement chirurgical de la cataracte et des lentilles intraoculaires.

Les personnes âgées, dont le cristallin a été remplacé par une lentille intraoculaire souffrent généralement de cyanopsie : ils ont tendance à voir les objets environnants en bleu, couleur complémentaire du jaune. Cette modification de la vision s'explique par le fait que le spectre de transmission du cristallin naturel change avec l'âge, de par un jaunissement dudit cristallin alors que la lentille intraoculaire intervenant en lieu et place dudit cristallin, vieilli i.e. jauni, est elle incolore. Pour remédier à cette modification de la vision, on a ainsi proposé, depuis quelques années, des lentilles intraoculaires jaunes.

De telles lentilles intraoculaires jaunes ont également été proposées en référence à d'éventuels dommages que serait susceptible de causer la lumière bleue (400-500 nm) à la rétine.

Dans le cadre de l'élaboration de ces lentilles intraoculaires colorées, tout comme dans celui de lentilles de contact colorées, de très nombreux paramètres entrent en jeu :
- la nature du colorant jaune en cause,
- la nature de la matrice au sein de laquelle il intervient,
- le mode d'intervention dudit colorant au sein de ladite matrice : simple dispersion ou réelle fixation (toute fixation est destinée à éviter l'élimination du colorant par élution),
- le type de réelle fixation en cause : la nature des fonctions chimiques mises en jeu, l'intervention ou non d'un groupe espaceur dans la structure chimique du colorant...

On vise bien évidemment à disposer, dans des conditions avantageuses d'obtention, de produits stables et performants.

Des produits de ce type ont été décrits dans la littérature brevets notamment par les sociétés Menikon, Hoya, Alcon et Canon-Staar.

La société Menikon a proposé :
- dans la demande JP-A-1 280 464 : des colorants solubles dont la formule chimique comporte un groupe polymérisable du type acryloyloxy, méthacryloyloxy, vinyle ou allyle, destinés à intervenir à titre de co-monomères dans l'élaboration de matrices polymères colorées ;
- dans la demande JP-A-2 232 056 : des composés polymérisables, à la fois colorants et filtres U.V, destinés également à intervenir dans des matrices polymères ;
- dans la demande EP-A-634 518 : un procédé pour produire des lentilles de contact teintées ;
- dans le brevet US-A-6,242,551 : des colorants polymérisables, destinés également à intervenir dans des matrices polymères.

La société Hoya a proposé :
- dans la demande EP-A-359 829 : un procédé pour produire des lentilles intraoculaires aptes à corriger la cyanopsie, lentilles intraoculaires dont le matériau constitutif renferme un colorant jaune, brun-jaune ou orange,
- dans la demande EP-A-799 864 : des colorants quinoniques polymérisables et les lentilles de contact souples les renfermant,
- dans la demande JP-A-10 195 324 : un colorant jaune, de la famille des pyrazolones, polymérisable et son intervention dans l'élaboration de lentilles intraoculaires souples par copolymérisation ;
- dans la demande EP-A-1 043 365 : des 1,2-dipyrazoéthènes polymérisables, convenant aussi à titre de colorants jaunes polymérisables, pour lentilles plastiques ophtalmiques.

La société Alcon a proposé :
- dans la demande EP-A-674 684 : d'autres colorants jaunes polymérisables (obtenus à l'issue d'une synthèse en deux étapes) et leur utilisation pour préparer des lentilles ophtalmiques. Lesdits colorants sont polymérisables de par la présence, dans leur formule, d'au moins un groupement polymérisable choisi parmi les groupements acryliques et méthacryliques. D'après l'enseignement de ce document, ces groupements de type acrylique se sont révélés plus performants que ceux de type vinylique.

La société Canon-Staar a enfin proposé :
- dans la demande US-A-2003/0078359 : deux nouvelles familles de colorants polymérisables, l'incorporation de tels colorants dans du matériau silicone et des lentilles intraoculaires renfermant lesdits colorants.

Dans un tel contexte, la Demanderesse propose présentement une nouvelle famille de colorants jaunes polymérisables.

Lesdits colorants, bifonctionnels, sont particulièrement intéressants, en termes de produits polymérisables, de par leur facilité de préparation, leur stabilité, leur réactivité, ainsi qu'en termes de colorants destinés à colorer des lentilles intraoculaires, de par leur couleur, apte à conférer auxdites lentilles un spectre UV/visible proche de celui du cristallin d'un homme à 25 ans. En effet, lesdits colorants sont aptes à conférer :
- une transmission quasi-nulle jusqu'à 400 nm,
- une augmentation constante de la transmission entre 400 et 500 nm, puis
- une transmission maximale dans les longueurs d'onde plus élevées (qui correspondent à des rayonnements non nocifs).

Selon son premier objet, la présente invention concerne donc de nouveaux composés, répondant à la formule (I) ci-après : 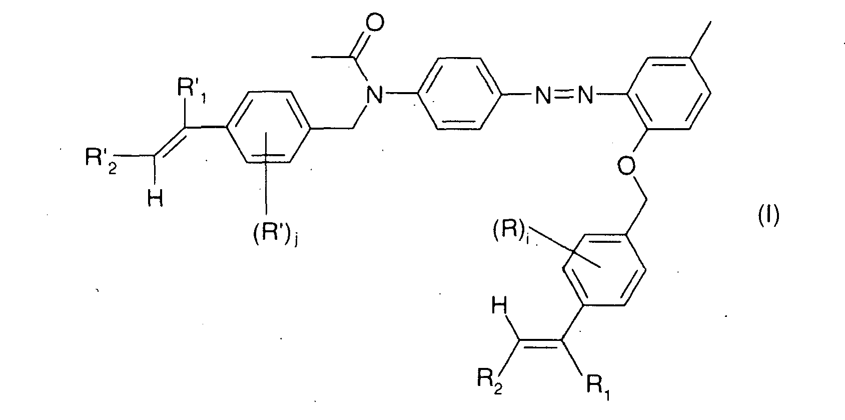 dans laquelle
R et R' sont, indépendamment, un groupe alkyle ou hydroxyalkyle linéaire comportant de 1 à 4 atomes de carbone ;
i et j sont, indépendamment, un nombre entier choisi parmi 0, 1, 2, 3 et 4 ; les différents R, lorsque i = 2, 3 ou 4, pouvant être identiques ou différents ; les différents R', lorsque j = 2, 3 ou 4, pouvant être identiques ou différents ; et
R₁, R₂, R'₁, R'₂ sont, indépendamment, l'hydrogène ou un groupe méthyle.

Lesdits nouveaux composés constituent les nouveaux colorants dont il est question ci-dessus.

Dans la formule (I) ci-dessus, lorsque i est différent de la valeur zéro, on a avantageusement R, alkyle ou hydroxyalkyle linéaire comportant 1 ou 2 atomes de carbone ; lorsque j est différent de la valeur zéro, on a avantageusement R', alkyle ou hydroxyalkyle linéaire comportant 1 ou 2 atomes de carbone.

Dans ladite formule (I) ci-dessus, on a avantageusement :
(R')ⱼ = (R)_{¡}
R'₁ = R₁, et
R'₂ = R₂.

Dans ladite formule (I) ci-dessus, on a très avantageusement i = j = 0 et R₁ = R₂ = R'₁ = R'₂ = H.

Le composé dé la formule (Ia) ci-dessous : 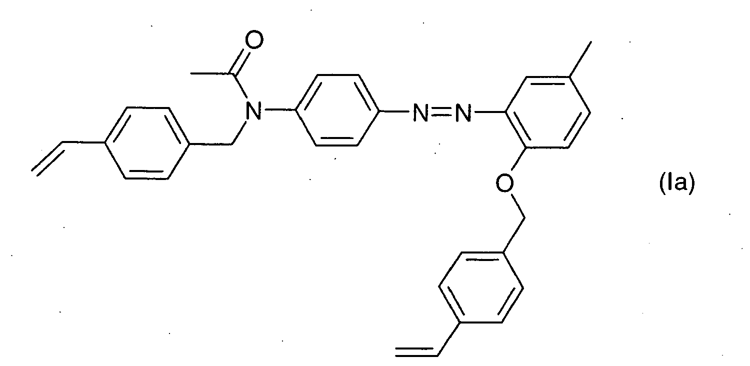 est ainsi particulièrement préféré.

Selon son second objet, la présente invention concerne le procédé de préparation des nouveaux composés de formule (I) ci-dessus. Il s'agit d'un procédé par analogie, d'un procédé d'éthérification de la fonction hydroxy et d'alkylation de la fonction amide du composé (le 4-(2-hydroxy-5-méthylphénylazo)acétanilide) de formule (II) ci-après :

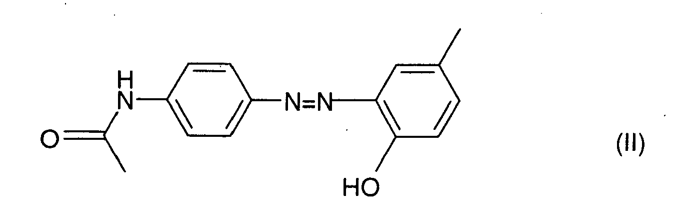

Ledit procédé d'éthérification et d'alkylation est mis en oeuvre en milieu solvant aprotique polaire alcalin. Il est mis en oeuvre en présence d'une base forte, telle KOH. Il est avantageusement mis en oeuvre dans le diméthylsulfoxyde (DMSO) ou le diméthylformamide (DMF).

Selon ledit procédé, ledit composé de formule (II) ci-dessus est mis à réagir avec au moins un dérivé styrénique de formule (III) : 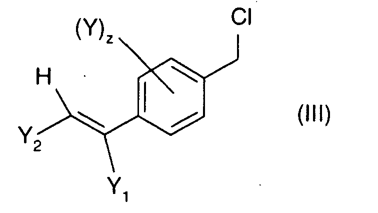 dans laquelle
Y est un groupe alkyle ou hydroxyalkyle linéaire comportant de 1 à 4 atomes de carbone ;
z est un nombre entier choisi parmi 0, 1, 2, 3, et 4 ; les différents Y lorsque z = 2, 3, ou 4, pouvant être identiques ou différents; et
Y₁ et Y₂ sont, indépendamment, l'hydrogène ou un groupe méthyle ;
ledit au moins un dérivé styrénique intervenant en excès stoechiométrique.

Lorsqu'un composé de formule (III) intervient, le composé de formule (I) obtenu présentent deux extrémités similaires.

Pour la préparation du composé préféré de formule (Ia), les fonctions hydroxy et amide du composé de formule (II) sont respectivement éthérifiées et alkylées par le 4-chlorométhylstyrène (unique composé de formule III à intervenir).

Lorsqu'au moins deux composés différents de formule (III) interviennent, on obtient un mélange de plusieurs composés de formule (I), des composés de formule (I) qui présentent deux extrémités similaires et des composés de formule (I) qui présentent des extrémités différentes. A partir de tels mélanges, on peut isoler, de façon connue *per se,* des composés d'une formule (I) donnée ...

Le dérivé styrénique de formule III (ou les dérivés styréniques de formule III, pris dans leur ensemble) interviennent toujours en excès stoechiométrique.

On vise ainsi à faire réagir les fonctions hydroxy et amide du composé de formule (II).

Généralement, ledit au moins un dérivé styrénique intervient en une quantité correspondant à au moins deux fois la quantité stoechiométrique et avantageusement en une quantité correspondant à au moins trois fois la quantité stoechiométrique.

La préparation des composés de formule (III), dérivés styréniques, ne pose pas de difficultés particulières à l'homme du métier.

Le composé de formule (II), répertorié sous le n° CAS 2832-40-8, est connu, comme indiqué ci-dessus, sous la dénomination C.I. Disperse Yellow 3. Il constitue un colorant jaune performant.

A partir d'un tel composé connu (colorant connu), le procédé de l'invention permet, en une seule étape (ce qui constitue un avantage certain par rapport au procédé selon l'enseignement de EP-A-674 684), d'obtenir les composés nouveaux de l'invention, de formule (I). Il s'agit de composés particulièrement intéressants, qui constituent eux-mêmes des colorants performants.

Selon son troisième objet, la présente invention concerne de tels colorants ; en d'autres termes, l'utilisation des composés de formule (I) à titre de colorants. Ces colorants nouveaux sont particulièrement intéressants et performants pour les raisons rappelées ci-après :
- leur synthèse ne comporte qu'une seule étape ;
- les fonctions éther et amide qui portent les groupements polymérisables ne sont pas clivables dans les conditions physiologiques ;
- la fonction polymérisable - fonction styrénique - est très réactive et génère, après réaction, des liaisons très stables ;
- lesdits colorants nouveaux ont conservé pratiquement la couleur du composé dont ils sont dérivés : le C.I. Disperse Yellow 3 (on observe par ailleurs un faible changement de couleur entre les compositions polymérisables et/ou réticulables qui contiennent le colorant sous forme dispersée et les matrices polymères qui contiennent ledit colorant lié de façon covalente. Les premières sont généralement d'un jaune brun tandis que les secondes sont généralement d'un jaune orangé) ;
- lesdits colorants permettent de préparer des matrices dont les spectres UV/visible sont proches de celui d'un homme de 25 ans.

Les autres objets de l'invention sont tous relatifs à l'utilisation des nouveaux composés de formule (I) à titre de colorants dans :
- des compositions polymérisables et/ou réticulables, et
- des matrices polymères colorées, susceptibles d'être obtenues à partir de telles compositions, notamment utiles comme matériau constitutif de lentilles intraoculaires.

Ainsi, selon son quatrième objet, la présente invention concerne-t-elle des compositions polymérisables et/ou réticulables par voie radicalaire qui comprennent, en mélange :
- des précurseurs d'une matrice polymère ; et
- une quantité efficace d'au moins un colorant de l'invention (un composé de formule (I)).

Il s'agit de toute composition susceptible d'être convertie en matrice au sein de laquelle le(s) colorant(s) a(ont) été piégé(s) et fixé(s) *via* au moins une de ses(leurs) fonctions polymérisables réactives.

Les précurseurs en cause peuvent être des monomères et les colorants de l'invention constituent, dans les mélanges, des co-monomères. Ce type de mélange est copolymérisé et généralement de surcroît réticulé pour générer une matrice polymère cohérente.

Les précurseurs en cause peuvent être des polymères et les colorants de l'invention sont alors fixés à leur squelette préalablement et/ou lors de leur réticulation.

Quels que soient les contextes exacts de l'intervention des colorants de l'invention, ils interviennent bien évidemment en quantité efficace pour conférer la couleur souhaitée au produit final, la couleur souhaitée à la composition, précurseur dudit produit final. La quantité efficace en cause est généralement comprise entre 0,02 et 0,15 % en poids du mélange (principalement : précurseurs de la matrice + colorant(s), en fait le mélange réactionnel).

Les mélanges en cause, appelés à réagir pour la génération d'une matrice au sein de laquelle le colorant est fixé, renferment généralement, de façon classique:
une quantité efficace d'au moins un initiateur de polymérisation ; et/ou
une quantité efficace d'au moins un agent de réticulation.

Ce type de réactifs intervient tout particulièrement dans un mélange de co-monomères à copolymériser.

A titre d'initiateur de copolymérisation radicalaire, par voie thermique, notamment de co-monomères de type acrylique, on préconise l'intervention :
- d'un mélange phosphite de sodium et phosphate de sodium (ou tout autre couple d'oxydoréduction) ;
- d'un composé azo, tel l'azobisisobutyronitrile (AIBN) ou le (2,2'-azobis (2,4-diméthyl valéronitrile)(AIVN), notamment commercialisé par la société WAKO sous la référence V65,
   dont on reproduit ci-après les formules développées :

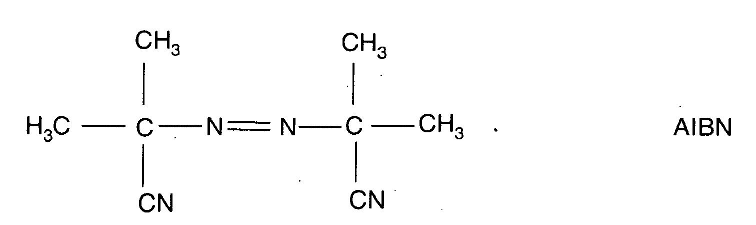

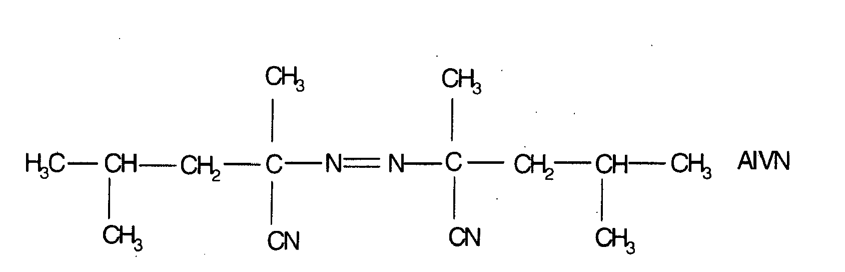

Ce dernier composé est particulièrement préféré au vu de sa faible toxicité, ainsi que de celle de ses produits de dégradation. (On note toutefois, de manière générale, que l'initiateur de polymérisation intervient en très faible quantité et se trouve généralement éliminé à l'issue du procédé de préparation des lentilles intraoculaires de l'invention) ; ou
- d'un peroxyde, tel le peroxyde de benzoyle.

L'homme du métier sait maîtriser la quantité d'intervention dudit initiateur de polymérisation radicalaire (généralement moins de 1 partie en poids, pour 100 parties en poids de monomères à copolymériser) et de manière générale, la cinétique de polymérisation du mélange réactionnel. Il sait notamment que, l'oxygène neutralisant l'action dudit initiateur de polymérisation, il est vivement préférable de l'éliminer du mélange réactionnel avant la montée en température. Un bullage de gaz inerte dudit mélange réactionnel est vivement préconisé. Pour ce qui concerne le programme de chauffe, son optimisation est à la portée de l'homme du métier.

On n'exclut pas, dans le cadre de l'invention, de préparer des compositions polymérisables par voie photochimique. On fait alors intervenir dans lesdites compositions une quantité efficace d'au moins un photo-initiateur conventionnel, de type benzophénone par exemple.

On a également parlé d'une quantité efficace d'au moins un agent de réticulation. Une telle réticulation peut s'avérer indispensable pour assurer la cohésion de la matrice et sa stabilité. Un agent de réticulation - au moins bifonctionnel - intervient donc généralement, en une quantité efficace, lors de la copolymérisation des co-monomères ou de la réticulation des polymères. Cette quantité efficace - généralement, au maximum de quelques parties en poids : en principe comprise entre 0,5 et 5 parties en poids, avantageusement comprise entre 0,5 et 2,5 parties en poids, pour 100 parties en poids de réactifs - doit évidemment rester raisonnable. Il ne s'agit pas par exemple que l'agent de réticulation intervenant constitue un "co-monomère" et modifie de façon conséquente les propriétés, notamment mécaniques, du copolymère attendu.

En tout état de cause, l'homme du métier n'ignore pas que l'augmentation du taux d'agent de réticulation intervenant diminue la teneur en eau des hydrogels et augmente leur température de transition vitreuse.

Pour ce qui concerne les fonctions réactives dudit agent de réticulation, il s'agit avantageusement de fonctions acrylates et/ou méthacrylates. L'homme du métier connaît de nombreux agents de réticulation, porteurs de telles fonctions, et notamment :
les diméthacrylate et diacrylate de butanediol,
les diméthacrylate et diacrylate d'hexanediol,
les diméthacrylate et diacrylate de décanediol,
le diméthacrylate d'éthylène glycol (EDMA),
le diméthacrylate de tétraéthylène glycol.

Dans le cadre de la présente invention, on préconise, de façon nullement limitative, l'intervention des agents de réticulation listés ci-dessus et tout particulièrement celle de l'EDMA.

Ainsi, les compositions polymérisables et/ou réticulables de l'invention renferment-elles généralement des agents de réticulation de ce type (ou d'un type équivalent) dont on trouve évidemment trace dans les matrices polymères obtenues à partir desdites compositions.

Dans un contexte de préparation de lentilles intraoculaires, l'homme du métier comprend que lesdites compositions sont également généralement susceptibles de renfermer une quantité efficace d'au moins un absorbeur d'ultra-violets. Ledit homme du métier n'ignore pas l'intérêt de stabiliser dans la structure de lentilles intraoculaires un composé de ce type qui jouera le rôle de filtre U.V. La stabilisation recherchée sera optimale si ledit absorbeur d'ultraviolets est lui aussi copolymérisable, i.e présente dans sa formule une fonction chimique réactive adéquate, du type double liaison, fonction acrylate ou méthacrylate.

L'homme du métier connaît de tels composés et plusieurs d'entre eux sont commercialement disponibles, notamment :
la 4-(2-acryloxyéthoxy)-2-hydroxy-benzophénone,
la 4-méthacryloxy-2-hydroxy benzophénone (MOBP),
le 1,3-bis-(4-benzoyl-3-hydroxyphénoxy)-2-propyl)acrylate,
le 2-(2'-méthacryloxy-5'-méthylphényl)benzotriazole.

Dans le cadre de la présente invention, tous ces composés conviennent mais la Demanderesse a plus particulièrement utilisé la MOBP dans la mesure où elle manipule depuis de nombreuses années ce filtre UV, pour la fabrication de lentilles intraoculaires.

Les filtres UV, interviennent généralement à raison de 0,25 à 5 parties en poids, avantageusement de 0,25 à 2 parties en poids, dans le mélange réactif.

Les compositions polymérisables et/ou réticulables de l'invention sont à base de précurseurs de matrice polymère renfermant une quantité efficace d'au moins un colorant de l'invention.

Avantageusement, lesdits précurseurs de matrice polymère sont choisi parmi les polysiloxanes et les monomères de type acrylique. Très avantageusement, ils sont choisis parmi les monomères de type acrylique.

Les polysiloxanes (réticulables) utilisés sont connus de l'homme du métier. Il y a en fait deux solutions à manipuler :
- la première contenant au moins un polysiloxane à terminaisons vinyliques et un catalyseur,
- la seconde contenant au moins un polysiloxane à terminaisons vinyliques et un polysiloxane avec des fonctions hydrure de silicium.

Lorsque ces deux solutions sont mises en présence, les fonctions 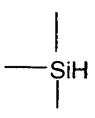 réagissent avec les fonctions 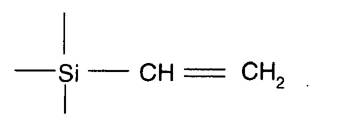 pour former une résine (matrice). Lorsque du colorant de l'invention est présent, il se trouve fixé et piégé au sein de ladite résine.

Les monomères de type acrylique en cause peuvent être de deux catégories : ils peuvent convenir pour générer une matrice hydrophile ou pour générer une matrice hydrophobe. De manière classique, on qualifie d'hydrophile une matrice avec des groupes hydroxyles qui permettent l'absorption d'eau par ladite matrice. Une telle matrice est aussi qualifiée d'hydrogel.

Dans le contexte de matrices hydrophiles, on préfère des mélanges précurseurs à base :
- de méthacrylate de 2-hydroxyéthyle (HEMA), et
- d'au moins un (méth)acrylate d'alkyle ou d'hydroxyalkyle en C1-C8.

On préfère tout particulièrement des mélanges du type :
- méthacrylate de 2-hydroxyéthyle (HEMA) et méthacrylate de méthyle (MMA), notamment ceux à partir desquels la société MENTOR avait réalisé des lentilles alors commercialisées sous la dénomination MEMORYLENS^{®} ;
- méthacrylate de 2-hydroxyéthyle (HEMA) et méthacrylate d'éthyle (EMA), notamment ceux décrits dans FR-A-2 757 065 ; ou
- méthacrylate de 2-hydroxyéthyle (HEMA) et méthacrylate de 6-hydroxyhexyle (HHMA), notamment ceux décrits dans US-A-5 217 491.

Dans le contexte des matrices hydrophobes, on préfère des mélanges précurseurs à base de monomères de formule : 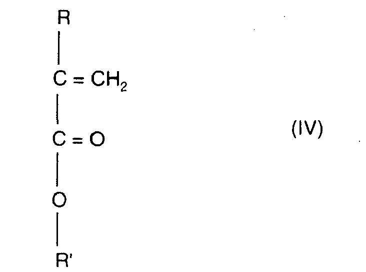 dans laquelle R est l'hydrogène ou un groupe méthyle et R' est un groupe alkyle linéaire ou ramifié comportant de 1 à 5 atomes de carbone ou un groupe (C1-C5-alkyl)phényle.

On préfère tout particulièrement des mélanges à base de méthacrylate de méthyle (MMA) ou à base d'acrylate d'éthyle (EA) et de méthacrylate d'éthyle (EMA)(notamment ceux décrits dans EP-A-674 684).

Selon son cinquième objet, la présente invention concerne les matrices polymères colorées susceptibles d'être obtenues par copolymérisation et/ou réticulation radicalaire des compositions polymérisables et/ou réticulables décrites ci-dessus. Lesdites matrices renferment, de façon caractéristique, le colorant jaune de formule (I). Ledit colorant jaune y est renfermé de façon stable. Il est partie prenante desdites matrices. Il a réagi, via sa(ses) double(s) liaison(s), pour entrer dans leur structure.

Les matrices colorées sont avantageusement de type polysiloxanes ou de type acrylique (voir ci-dessus).

Selon son dernier objet, la présente invention concerne enfin des lentilles intraoculaires constituées en totalité ou en partie de matrices polymères colorées de l'invention. Au moins l'optique desdites lentilles est ainsi constituée de matrices polymères colorées de l'invention. Avantageusement l'optique et l'haptique desdites lentilles sont constituées de matrices polymères colorées de l'invention.

De telles lentilles sont particulièrement performantes au vu des avantages énoncés en amont dans le présent texte pour les composés de formule (I), intervenant à titre de colorant jaune en leur sein.

L'invention est illustrée par les exemples ci-après et les figures annexées.

On a tout d'abord préparé un colorant jaune polymérisable selon l'invention - le composé de formule (Ia) - puis on l'a incorporé dans des compositions polymérisables et réticulables qui ont été polymérisées et réticulées pour générer des matrices polymères colorées convenant parfaitement à titre de matériau constitutif de lentilles intraoculaires.

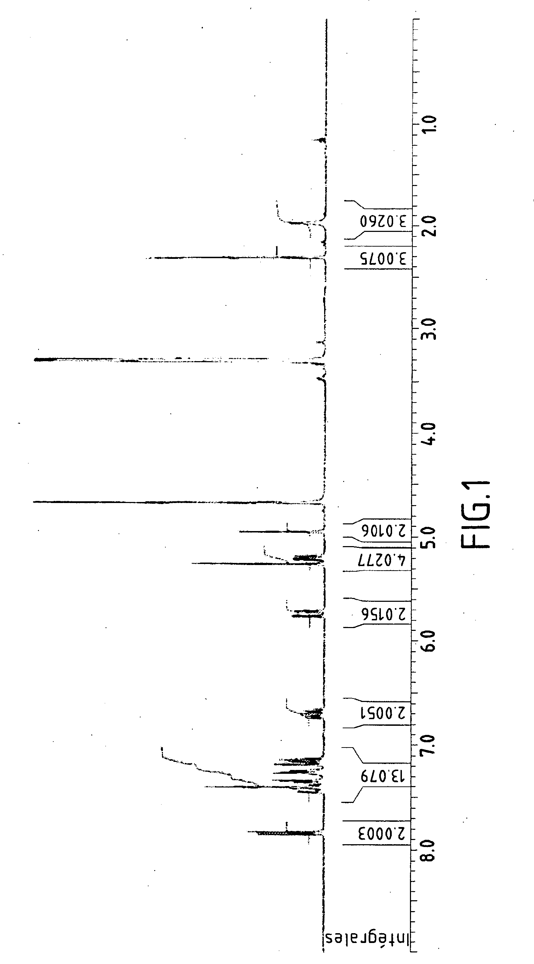

Sur la figure 1, on montre le spectre RMN ¹H dudit composé de formule (Ia), tel que préparé. Sur ledit spectre, on retrouve les pics caractéristiques :
- des cycles aromatiques entre 7 et 8 ppm,
- des liaisons vinyliques à 6,70 , 5,75 et 5,20 ppm,
- de CH₂-N à 5,25 ppm
- de CH₂-Ar à 4,95 ppm
- de CH₃-Ar à 2,31 ppm et
- de CH₃-CO-N à 1,97 ppm.

Sur la figure 2, on montre des spectres de transmission. Ladite figure 2 est commentée ci-après.

### Exemple

### Préparation du composé de formule (Ia) :

Ladite préparation est mise en oeuvre à température ambiante.

1 g de C.I. Disperse Yellow 3 (n° CAS 2832-40-8) est introduit dans un ballon puis recouvert de 50 ml de diméthylformamide. L'ensemble est agité pendant 1 h. L'ajout de 0,32 g de potasse entraîne la coloration de la solution en violet. L'agitation est ensuite maintenue pendant 40 min.

Du 4-chlorométhylstyrène (0,48 ml) est alors ajouté. Le milieu réactionnel est agité pendant 42 h.

A l'issue de ces 42 h, 100 ml d'eau déionisée sont ajoutés audit milieu réactionnel.

Le monomère (composé de formule (Ia)) est extrait à l'éther. La phase organique est ensuite lavée avec 5 fois 100 ml d'eau déionisée, puis séchée sur sulfate de sodium, le solvant est évaporé.

Le monomère est recristallisé dans l'éther préalablement séché sur sulfate de sodium.

### Préparation de matrices colorées hydrophiles

Le composé de formule (Ia) est pesé dans un flacon en verre puis est ajoutera une solution de monomères, renfermant :
- 82,5 g de méthacrylate de 2-hydroxyéthyle (HEMA),
- 17,5 g de méthacrylate d'éthyle (EMA),
- 0,8 g de diméthacrylate d'éthylèneglycol, et
- 0,5 g de 4-méthacryloxy-2-hydroxybenzophénone,
de telle sorte que la concentration en colorant selon les échantillons soit comprise entre 0,02 et 0,15 % en poids.

Du peroxyde de benzoyle (0,2 g) est ensuite ajouté.

Le mélange est homogénéisé aux ultrasons et dégazé à l'argon. La polymérisation (réticulation) est ensuite effectuée à 40°C pendant 96 h. Le durcissement ultérieur des palets est assuré par une phase de post-polymérisation thermique de 3 heures à 100°C.

L'efficacité de la réaction de polymérisation (réticulation), plus précisément celle du piégeage du colorant a été évaluée de la manière précisée ci-après.

Des pastilles hydratées ont été extraites, pendant un minimum de 6 heures, par un mélange isovolumique de méthanol et d'eau distillée. On a comparé les spectres UV-visible desdites pastilles avant et après extraction.

La perte d'absorbance desdites pastilles est restée inférieure à 10 % entre 400, et 500 nm, a atteint 20 % pour les faibles transmissions entre 350 et 400 nm. Une telle perte d'absorbance est peu gênante.

Le matériau obtenu offre par ailleurs un bon pouvoir filtrant des rayonnements bleus, voisin de celui obtenu par le cristallin d'un homme de 25 ans et une très bonne transmission pour les longueurs d'ondes au-delà du bleu.

### Préparation d'autres matrices colorées hydrophile

Le composé de formule (Ia) est pesé dans un flacon en verre puis est ajouté à une solution de monomères, renfermant :
- 82,5 g de méthacrylate de 2-hydroxyéthyle (HEMA),
- 17,5 g de méthacrylate de méthyle (MMA),
- 0,8 g de diméthacrylate d'éthylèneglycol, et
- 0,5 g de 4-méthacryloxy-2-hydroxybenzophénone,
de telle sorte que la concentration en colorant selon les échantillons soit comprise entre 0,02 et 0,15 % en poids.

Du peroxyde de benzoyle (0,2 g) est ensuite ajouté.

Le mélange est homogénéisé aux ultrasons et dégazé à l'argon. La polymérisation (réticulation) est ensuite effectuée à 40°C pendant 96 h. Le durcissement ultérieur des palets est assuré par une phase de post-polymérisation thermique de 3 heures à 100°C.

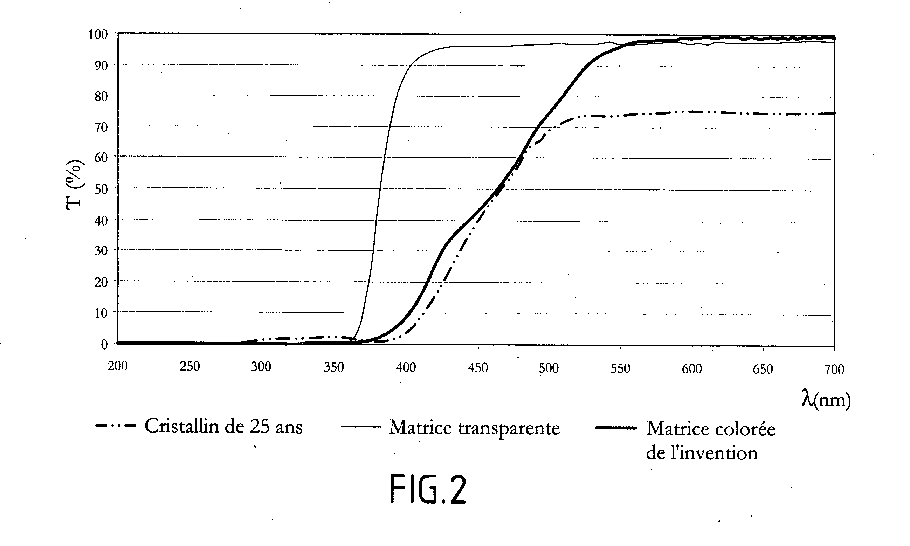

Le matériau obtenu offre un bon pouvoir filtrant des rayonnements bleus, voisin de celui obtenu par le cristallin d'un homme de 25 ans et une très bonne transmission pour les longueurs d'ondes au-delà du bleu. Ceci est montré sur la figure 2 annexée.

Sur ladite figure 2 on montre trois spectres de transmission, T= f (λ):
- le premier (- -- -) correspondant au cristallin d'un homme de 25 ans,
- le troisième (-) à une matrice colorée (HEMA + MMA + colorant de l'invention à une teneur de 0,075% en poids), telle que préparée ci-dessus;
- le second (-) à la matrice transparente correspondante (HEMA +MMA), préparée de la même façon sans intervention de colorant.

### Préparation de matrices polymères colorées hydrophobes

Le composé de formule (la) est pesé dans un flacon en verre puis est ajouté à une solution de monomères, renfermant :
- 100 g de méthacrylate de méthyle (MMA),
- 0,8 g de diméthacrylate d'éthylèneglycol, et
- 0,5 g de 4-méthacryloxy-2-hydroxybenzophénone,
de telle sorte que la concentration en colorant selon les échantillons soit comprise entre 0,02 et 0,15 % en poids.

Du peroxyde de benzoyle (0,2 g) est ensuite ajouté.

Le mélange est homogénéisé aux ultrasons et dégazé à l'argon. La polymérisation (réticulation) est ensuite effectuée à 40°C pendant 96 h. Le durcissement ultérieur des palets est assuré par une phase de post-polymérisation thermique de 3 heures à 100°C.

Le matériau obtenu offre un bon pouvoir filtrant des rayonnements bleus, voisin de celui obtenu par le cristallin d'un homme de 25 ans et une très bonne transmission pour les longueurs d'ondes au-delà du bleu.

## Revendications

1. Composés de formule: 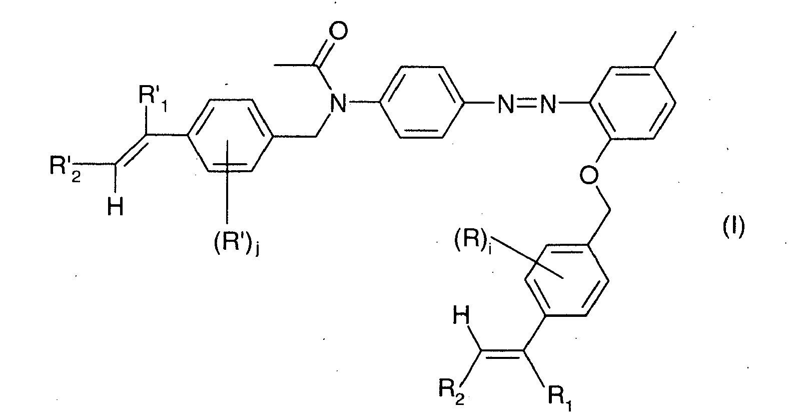 dans laquelle
R et R' sont, indépendamment, un groupe alkyle ou hydroxyalkyle linéraire comportant de 1 à 4 atomes de carbone ;
i et j sont, indépendamment, un nombre entier choisi parmi 0, 1, 2, 3 et 4 ; les différents R, lorsque i = 2, 3 ou 4, pouvant être identiques ou différents ; les différents R', lorsque j = 2, 3 ou 4, pouvant être identiques ou différents ; et
R₁, R₂, R'₁, R'₂ sont, indépendamment, l'hydrogène ou un groupe méthyle.

2. Composés selon la revendication 1, de formule (I) dans laquelle :
(R')ⱼ = (R)ᵢ
R'₁ = R₁, et
R'₂ = R₂.

3. Composé selon la revendication 1 ou 2, de formule :

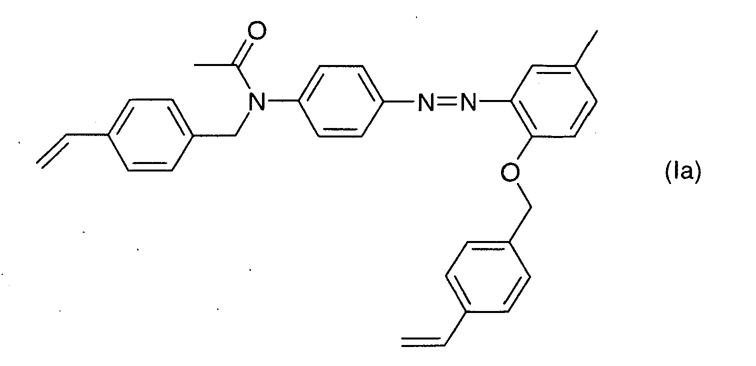

4. Procédé pour la préparation d'un composé selon l'une quelconque des revendications 1 à 3, **caractérisé en ce qu'**il comprend, en milieu solvant aprotique polaire alcalin, la réaction du composé de formule (II) : 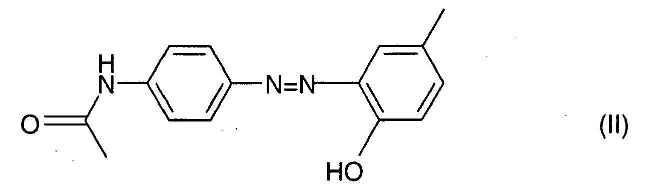 avec au moins un dérivé styrénique de formule (III) : 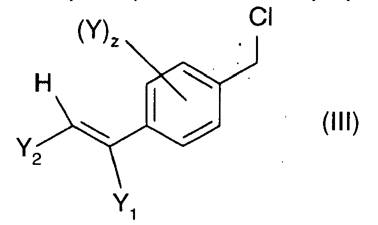 dans laquelle
Y est un groupe alkyle ou hydroxyalkyle linéaire comportant de 1 à 4 atomes de carbone ;
z est un nombre entier choisi parmi 0, 1, 2, 3, et 4 ; les différents Y, lorsque z = 2, 3, ou 4, pouvant être identiques ou différents; et
Y₁ et Y₂ sont, indépendamment, l'hydrogène ou un groupe méthyle ;
ledit au moins un dérivé styrénique intervenant en excès stoechiométrique.

5. Procédé selon la revendication 4, **caractérisé en ce que** ledit au moins un composé de formule (III) intervient en une quantité correspondant à au moins deux fois la quantité stoechiométrique, avantageusement à au moins trois fois ladite quantité stoechiométrique.

6. Procédé selon la revendication 4 ou 5, **caractérisé en ce qu'**il comprend la réaction dudit composé de formule (II) avec le 4-chlorométhylstyrène.

7. Colorant jaune polymérisables consistant en un composé selon l'une quelconque des revendications 1 à 3.

8. Composition polymérisable et/ou réticulable par voie radicalaire, **caractérisée en ce qu'**elle comprend un mélange :
- de précurseurs d'une matrice polymère ; et
- d'une quantité efficace d'au moins un colorant jaune polymérisable selon la revendication 7 ; ladite quantité efficace représentant généralement 0,02 à 0,15 % en poids dudit mélange.

9. Composition selon la revendication 8, **caractérisée en ce que** ledit mélange renferme en outre une quantité efficace d'au moins un initiateur de polymérisation et/ou une quantité efficace d'au moins un agent de réticulation.

10. Composition selon la revendication 8 ou 9, **caractérisée en ce que** ledit mélange renferme en outre une quantité efficace d'au moins un absorbeur d'ultraviolets, avantageusement copolymérisable.

11. Composition selon l'une quelconque des revendications 8 à 10, **caractérisée en ce que** lesdits précurseurs d'une matrice polymère sont choisis parmi les polysiloxanes et les monomères de type acrylique.

12. Composition selon l'une quelconque des revendications 8 à 11, **caractérisée en ce que** lesdits précurseurs sont des monomères de type acrylique qui conviennent pour générer une matrice hydrophile.

13. Composition selon la revendication 12, **caractérisée en ce que** les monomères de type acrylique consistent en du méthacrylate de 2-hydroxyéthyle (HEMA) et au moins un (méth)acrylate d'alkyle ou d'hydroxyalkyle en C1-C8.

14. Composition selon la revendication 12 ou 13, **caractérisée en ce que** lesdits monomères de type acrylique consistent en des mélanges :
- méthacrylate de 2-hydroxyéthyle (HEMA) et méthacrylate de méthyle (MMA) ;
- méthacrylate de 2-hydroxyéthyle (HEMA) et méthacrylate d'éthyle (EMA) ; ou
- méthacrylate de 2-hydroxyéthyle (HEMA) et méthacrylate de 6-hydroxyhexyle (HHMA).

15. Composition selon l'une quelconque des revendications 8 à 11, **caractérisée en ce que** lesdits précurseurs sont des monomères de type acrylique qui conviennent pour générer une matrice hydrophobe.

16. Composition selon la revendication 15, **caractérisée en ce que** lesdits monomères de type acrylique répondent à la formule : 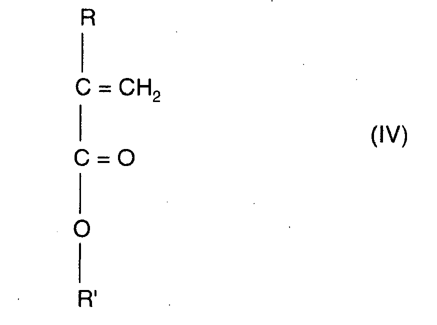 dans laquelle R est l'hydrogène ou un groupe méthyle et R' est un groupe alkyle linéaire ou ramifié comportant de 1 à 5 atomes de carbone ou un groupe (C1-C5 alkyl)phényle.

17. Matrice polymère colorée susceptible d'être obtenue par copolymérisation et/ou réticulation radicalaire d'une composition polymérisable et/ou réticulable selon l'une quelconque des revendications 8 à 16.

18. Lentilles intraoculaires présentant une optique et une haptique, **caractérisées en ce que** ladite optique (ladite optique et ladite haptique) est(sont) en une matrice polymère colorée selon la revendication 17.

## Claims

1. Compounds of the formula 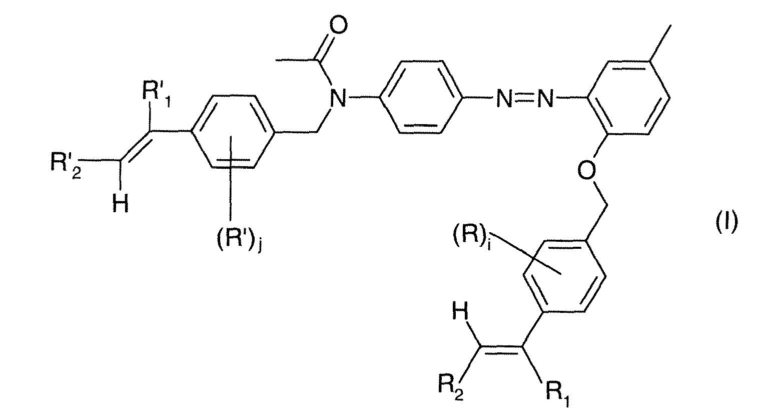 in which
R and R' independently are a linear alkyl or hydroxyalkyl group containing from 1 to 4 carbon atoms;
i and j independently are an integer selected from 0, 1, 2, 3 and 4, it being possible for the different R, when i = 2, 3 or 4, to be identical or different, and it being possible for the different R', when j = 2, 3 or 4, to be identical or different; and
R₁, R₂ R'₁ and R'₂ independently are hydrogen or a methyl group.

2. The compounds according to claim 1 of formula (I) in which
(R')ⱼ = (R)ᵢ,
R'₁ =R₁, and
R'2 = R₂.

3. Compound according to claim 1 or 2 of the formula

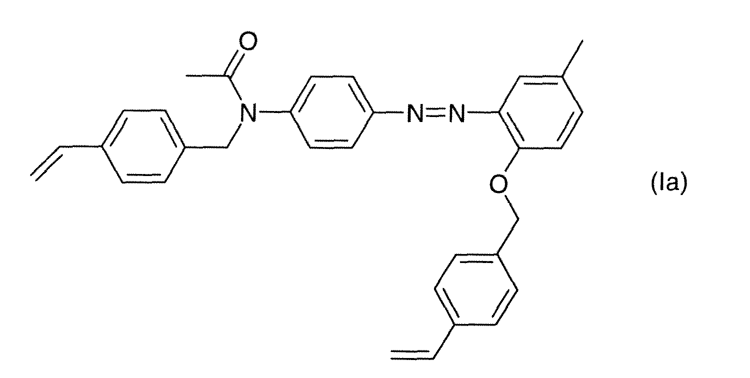

4. Process for the preparation of a compound according to any one of claims 1 to 3, **characterized in that** it comprises reacting the compound of formula (II) : 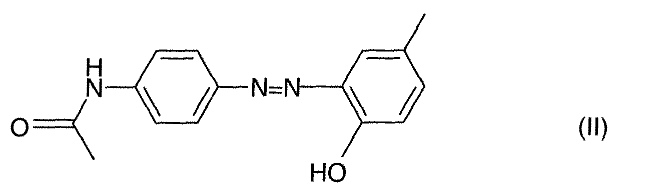 in an alkaline, polar, aprotic solvent medium, with at least one styrene derivative of formula (III): 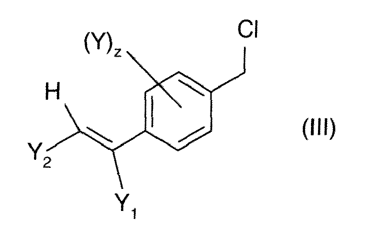 in which
Y is a linear alkyl or hydroxyalkyl group containing from 1 to 4 carbon atoms;
z is an integer selected from 0, 1, 2, 3 and 4, it being possible for the different Y, when z = 2, 3 or 4, to be identical or different; and
Y₁ and Y₂ independently are hydrogen or a methyl group,
said at least one styrene derivative being used in stoichiometric excess.

5. The process according to claim 4, **characterized in that** said at least one compound of formula (III) is used in an amount to at least twice the stoichiometric amount and advantageously at least three times said stoichiometric amount.

6. The process according to claim 4 or 5, **characterized in that** it comprises reacting said compound of formula (II) with 4-chloromethylstyrene.

7. Polymerizable yellow dye consisting of a compound according to anyone of claims 1 to 3.

8. Composition which is polymerizable and/or crosslinkable by a free-radical process, **characterized in that** it comprises a mixture of:
- precursors of a polymer matrix; and
- an effective amount of at least one polymerizable yellow dye according to claim 7, said effective amount generally representing 0.02 to 0.15% by weight of said mixture.

9. The composition according to claim 8, **characterized in that** said mixture also contains an effective amount of at least one polymerization initiator and/or an effective amount of at least one crosslinking agent.

10. The composition according to claim 8 or 9, **characterized in that** said mixture also contains an effective amount of at least one ultraviolet absorber that is advantageously copolymerizable.

11. The composition according to any one of claims 8 to 10, **characterized in that** said polymer matrix precursors are selected from polysiloxanes and monomers of acrylic type.

12. The composition according to any one of claims 8 to 11, **characterized in that** said precursors are monomers of acrylic type that are suitable for generating a hydrophilic matrix.

13. The composition according to claim 12, **characterized in that** the monomers of acrylic type consist of 2-hydroxyethyl methacrylate (HEMA) and at least one C1-C8 alkyl or hydroxyalkyl (meth)acrylate.

14. The composition according to claim 12 or 13, **characterized in that** said monomers of acrylic type consist of the following mixtures:
- 2-hydroxyethyl methacrylate (HEMA) and methyl methacrylate (MMA);
- 2-hydroxyethyl methacrylate (HEMA) and ethyl methacrylate (EMA); or
- 2-hydroxyethyl methacrylate (HEMA) and 6-hydroxyhexyl methacrylate (HHMA).

15. The composition according to anyone of claims 8 to 11, **characterized in that** said precursors are monomers of acrylic type that are suitable for generating a hydrophobic matrix.

16. The composition according to claim 15, **characterized in that** said monomers of acrylic type have the formula 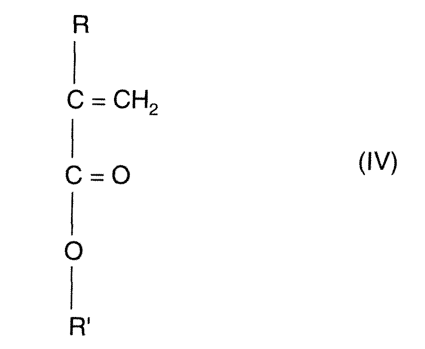 in which R is hydrogen or a methyl group and R' is a linear or branched alkyl group containing from 1 to 5 carbon atoms or a (C1-C5 alkyl)phenyl group.

17. Colored polymer matrix obtainable by free-radical copolymerization and/or crosslinking of a polymerizable and/or crosslinkable composition according to any one of claims 8 to 16.

18. Intraocular lenses possessing optics and haptics, **characterized in that** said optics (said optics and said haptics) are made up of a colored polymer matrix according to claim 17.

## Patentansprüche

1. Verbindungen mit der Formel: 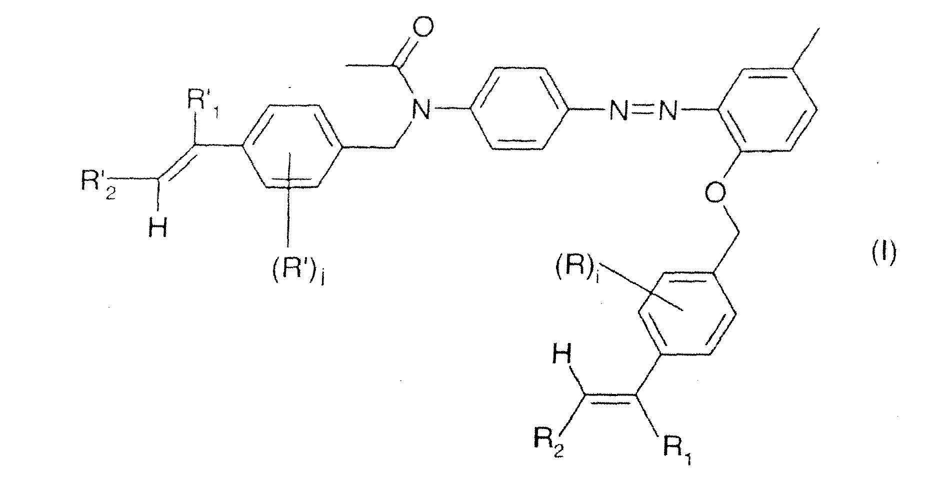 worin
R und R' unabhängig für eine lineare Alkyl- oder Hydroalkylgruppe stehen, die 1 bis 4 Kohlenstoffatome umfaßt,
i und j unabhängig für eine ganze Zahl stehen, ausgewählt aus 0, 1, 2, 3 und 4, die verschiedenen R, wenn i = 2, 3 oder 4, gleich oder verschieden sein können, die verschiedenen R', wenn j = 2, 3 oder 4, gleich oder verschieden sein können, und R₁, R₂, R'₁, R'₂ unabhängig für Wasserstoff oder eine Methylgruppe stehen.

2. Verbindungen nach Anspruch 1 mit der Formel (I), worin:
(R')ⱼ = (R)ᵢ
R'₁ = R₁ und
R'₂ = R₂.

3. Verbindungen nach Anspruch 1 oder 2 mit der Formel:

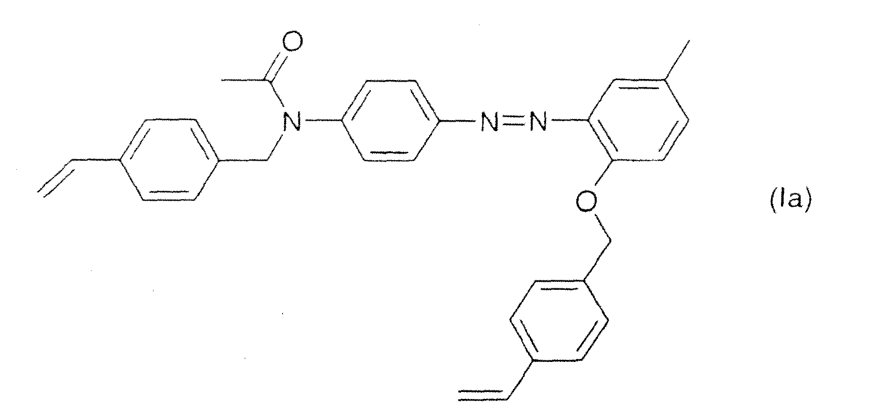

4. Verfahren zur Herstellung einer Verbindung nach irgendeinem der Ansprüche 1 bis 3, **dadurch gekennzeichnet, daß** es, in einem alkalischen polaren aprotischen Lösemittelmedium, das Umsetzen der Verbindung mit der Formel (II): 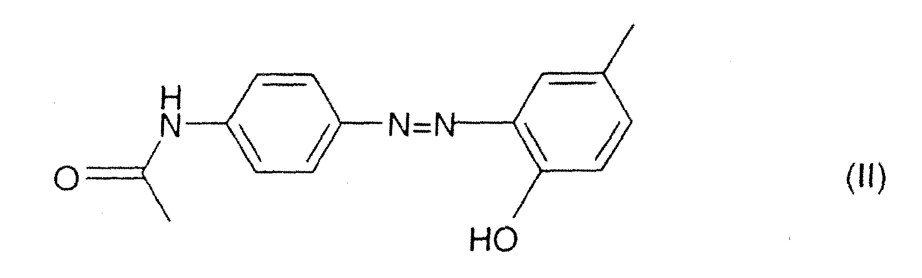 mit mindestens einem Styrenderivat mit der Formel (III): 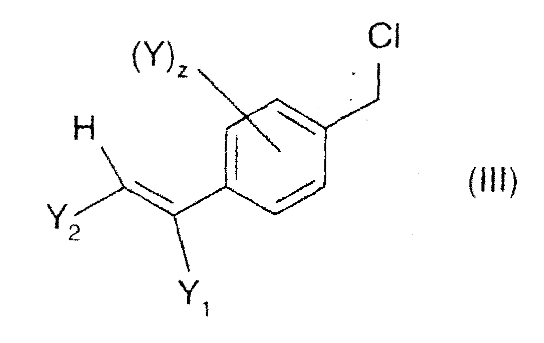 umfaßt,
worin
Y für eine lineare Alkyl- oder Hydroalkylgruppe steht, die 1 bis 4 Kohlenstoffatome umfaßt,
z für eine ganze Zahl steht, ausgewählt aus 0, 1, 2, 3 und 4, die verschiedenen Y, wenn z = 2, 3 oder 4, gleich oder verschieden sein können, und
Y₁ und Y₂ unabhängig für Wasserstoff oder eine Methylgruppe stehen,
wobei das mindestens eine Styrenderivat in stöchiometrischem Überschuß verwendet wird.

5. Verfahren nach Anspruch 4, **dadurch gekennzeichnet, daß** die mindestens eine Verbindung mit der Formel (III) in einer Menge verwendet wird, die mindestens zwei Mal der stöchiometrischen Menge, vorteilhafterweise mindestens drei Mal der stöchiometrischen Menge entspricht.

6. Verfahren nach Anspruch 4 oder 5, **dadurch gekennzeichnet, daß** es das Umsetzen der Verbindung mit der Formel (II) mit 4-Chlormethylstyren umfaßt.

7. Polymerisierbarer gelber Farbstoff, der aus einer Verbindung nach irgendeinem der Ansprüche 1 bis 3 besteht.

8. Radikalisch polymerisierbare und/oder vernetzbare Zusammensetzung, **dadurch gekennzeichnet, daß** sie als Gemisch umfaßt:
- Vorläufer einer Polymermatrix, und
- eine wirksame Menge mindestens eines polymerisierbaren gelben Farbstoffs nach Anspruch 7, wobei die wirksame Menge im Allgemeinen 0,02 bis 0,15 Gew.-% des Gemischs darstellt.

9. Zusammensetzung nach Anspruch 8, **dadurch gekennzeichnet, daß** das Gemisch ferner eine wirksame Menge mindestens eines Polymerisationsinitiators und/oder eine wirksame Menge mindestens eines Vernetzungsmittels beinhaltet.

10. Zusammensetzung nach Anspruch 8 oder 9, **dadurch gekennzeichnet, daß** das Gemisch ferner eine wirksame Menge mindestens eines UV-Absorbers beinhaltet, der vorteilhafterweise copolymerisierbar ist.

11. Zusammensetzung nach irgendeinem der Ansprüche 8 bis 10, **dadurch gekennzeichnet, daß** die Vorläufer einer Polymermatrix aus Polysiloxanen und Monomeren des Acryltyps ausgewählt sind.

12. Zusammensetzung nach irgendeinem der Ansprüche 8 bis 11, **dadurch gekennzeichnet, daß** die Vorläufer Monomere des Acryltyps sind, die geeignet sind, um eine hydrophile Matrix zu erzeugen.

13. Zusammensetzung nach Anspruch 12, **dadurch gekennzeichnet, daß** die Monomere des Acryltyps aus 2-Hydroxyethylmethacrylat (HEMA) und mindestens einem C1-C8-Alkyl- oder Hydroxyalkyl(meth)acrylat bestehen.

14. Zusammensetzung nach Anspruch 12 oder 13, **dadurch gekennzeichnet, daß** die Monomere des Acryltyps aus Gemischen aus:
- 2-Hydroxyethylmethacrylat (HEMA) und Methylmethacrylat (MMA),
- 2-Hydroxyethylmethacrylat (HEMA) und Ethylmethacrylat (EMA), oder
- 2-Hydroxyethylmethacrylat (HEMA) und 6-Hydroxyhexylmethacrylat (HHMA), bestehen.

15. Zusammensetzung nach irgendeinem der Ansprüche 8 bis 11, **dadurch gekennzeichnet, daß** die Vorläufer Monomere des Acryltyps sind, die geeignet sind, um eine hydrophobe Matrix zu erzeugen.

16. Zusammensetzung nach Anspruch 15, **dadurch gekennzeichnet, daß** die Monomere des Acryltyps der folgenden Formel entsprechen: 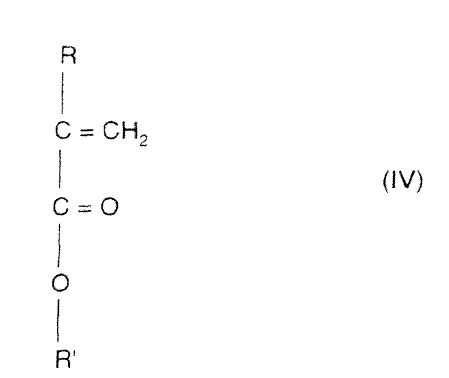 worin R für einen Wasserstoff oder eine Methylgruppe steht und R' für eine lineare oder verzweigte Alkylgruppe, die 1 bis 5 Kohlenstoffatome umfaßt, oder eine (C1-C5-Alkyl)phenylgruppe steht.

17. Farbige Polymermatrix, die durch radikalische Copolymerisation und/oder Vernetzung einer polymerisierbaren und/oder vernetzbaren Zusammensetzung nach irgendeinem der Ansprüche 8 bis 16 erhalten werden kann.

18. Intraokulare Linsen, die eine Optik und eine Haptik aufweisen, **dadurch gekennzeichnet, daß** die Optik (die Optik und die Haptik) eine farbige Polymermatrix nach Anspruch 17 ist (sind).
